# FASCICULE DE BREVET EUROPEEN

(11) **EP 1 871 332 B1**
(45) Date de publication et mention de la délivrance du brevet: **15.02.2012**
(21) Numéro de dépôt: 06755449.3
(22) Date de dépôt: 21.04.2006
(51) Int. Cl.: A61K 8/97, A61Q 19/08, A61Q 17/00, A61Q 19/00

(54) **Composition cosmétique pour lutter contre les conséquences cutanées de la pollution**
Kosmetische Zusammensetzung zur Bekämpfung von Folgen der Luftverschmutzung für die Haut
Cosmetic composition for combating cutaneous consequences of pollution

(30) Priorité: 22.04.2005 FR 0551037
(43) Date de publication de la demande: 02.01.2008
(73) Titulaire: Laboratoires Clarins, 92200 Neuilly sur Seine (FR)
(72) Inventeur: COURTIN, Olivier, F-92100 Boulogne-Billancourt (FR)
(74) Mandataire: Novagraaf Technologies
(86) Numéro de dépôt international: PCT/FR2006/000896
(87) Numéro de publication internationale: WO 2006/111666

(56) Documents cités:
- EP-A2- 1 250 930
- WO-A-98/44905
- FR-A- 2 842 102
- US-A1- 2004 175 351

## Description

La présente invention concerne une nouvelle composition cosmétique capable de lutter et de prévenir les effets de la pollution atmosphérique sur la peau contenant deux extraits végétaux : *Camellia sinensis* et *Lampsana communis.*

La peau d'une surface de 2m² environ est le plus grand organe de l'organisme. Indispensable à la vie, véritable interface entre le corps et l'environnement, c'est une barrière interactive. Elle possède de grandes capacités d'adaptation ou de réactions face aux agressions. Elle est aussi un site privilégié d'absorption des polluants de l'environnement, avec passage de substances pouvant avoir un effet toxique cutané direct.

L'air pur est un mélange de 78% d'azote, 21% d'oxygène, 0.09% d'argon, 0.1% de vapeur d'eau et d'autres gaz plus ou moins rares dont le gaz carbonique, l'hydrogène, l'ozone. La pollution atmosphérique se définit par la modification de l'air pur, par modification de ses composants, ou par adjonction d'éléments nocifs. Très schématiquement, il est possible de considérer que les polluants actuellement répertoriés sont essentiellement produits par l'industrie, le chauffage et la circulation automobile. Il est habituel de les classer en sept familles chimiques :
- les agents oxydants tels que l'ozone ou les oxydes d'azote sont irritants et générateurs de radicaux libres.
- les poussières sont des particules en suspension fixant parfois des hydrocarbures polycycliques. Leur rôle nocif est modifié par la température et le degré d'humidité de l'air, les particules acides sont irritantes ; on observe une baisse de l'hydratation et de l'oxygénation des tissus.
- les produits chimiques organiques sont cancérigènes, ils proviennent des déchets de l'industrie et de la combustion des automobiles.
- le monoxyde de carbone entraîne une hypoxie tissulaire, il provient à 80% des automobiles.
- les hydrocarbures et les solvants représentent 50% de la pollution automobile, ils sont toxiques, irritants, cancérigènes et mutagènes. En présence de lumière, ils réagissent avec les oxydes d'azote et produisent de l'ozone.
- le dioxyde de soufre est l'un des produits de la combustion du mazout et du charbon, il provoque une altération du film hydro-lipidique et des irritations.
- les métaux tels que le plomb, le zinc, l'aluminium, le mercure, ... peuvent interférer avec le métabolisme cellulaire, par atteinte des réactions enzymatiques. Ils participent aux dommages oxydatifs avec lésions de l'ADN et des lipides cellulaires.

Pour tous ces polluants, les risques varient selon leur concentration, la durée d'exposition, leurs associations, mais surtout de la prédisposition du sujet. Ils présentent entre eux une synergie d'action.

Les effets de la pollution sur la peau sont variés : on observe une acidification du pH cutané, une baisse de l'hydratation avec augmentation de la perte transépidermique en eau, une augmentation de la desquamation, une diminution de la souplesse cornée, une modification des lipides de surface par action des radicaux libres, une baisse du métabolisme énergétique cellulaire. La répétition de ces agressions initie un processus inflammatoire et prédispose aux réactions d'intolérance.

Ainsi une composition cosmétique capable de lutter et de prévenir les effets de la pollution atmosphérique sur la peau doit pouvoir lutter contre ces divers phénomènes.

WO 98/44905 décrit des compositions cosmétiques contenant un extrait de *Lampsana communis* qui possède des propriétés protectrices de la peau contre les radicaux libres.

FR 2842102 décrit l'utilisation des extrait de thé blanc *(Camellia sinensis)* de la variété Paï Mu Tan dans des compositions cosmétiques à cause de ses propriétés anti-radicalaires et anti-oxydantes.

US 2004/175351 décrit l'utilisation des extraits de thé blanc contre les processus oxydatifs responsables du vieillissement de la peau.

EP 1250930 divulgue des mélanges d'extraits végétaux comme activateurs de la synthèse de l'ATP.

Les travaux de la demanderesse ont permis de mettre en évidence que l'association de deux extraits végétaux de *Camellia sinensis* et *Lampsana communis* présentait un effet protecteur des effets de la pollution au niveau cutané.

L'invention concerne donc, tout particulièrement, l'utilisation d'une composition contenant un extrait de *Camellia sinensis* et de *Lampsana communis,* pour augmenter la vitesse de synthèse d'ATP basale et mitochondriale des cellules de la peau, ladite composition contenant de 0,025 à 5 % en poids d'extrait de *Camellia sinensis* et d'extrait de *Lampsana communis.*

Le *Camellia sinensis* appelé aussi thé blanc est originaire du sud-est asiatique, la variété utilisée dans la composition selon l'invention est le Paï Mu Tan. Il appartient à la famille des théacées au même titre que le thé vert ou le thé noir. Il se distingue de ces autres qualités par sa préparation qui se limite à des opérations minimalistes lui permettant ainsi de conserver sa forte teneur en polyphénols.

L'extrait de *Camellia sinensis* utilisé dans la composition selon l'invention est obtenu par extraction hydro-glycolique des feuilles de thé blanc. On obtient ainsi une solution translucide de couleur orange foncé et d'odeur caractéristique. Cette solution est titrée en polyphénols totaux et en OPC (olygomères proanthocyanidiques) ; il présente les caractéristiques analytiques suivantes :
- extrait sec 2 à 4%
- pH 5.3 à 7.3
- dosage des polyphénols totaux : > 5g/l équivalent acide gallique
- dosage des OPC : 2.5 g/l équivalent cathéchine.

Le *Lampsana communis,* de la famille des astéracées, est une plante annuelle très répandue, poussant au bord des routes et autres sites soumis à la pollution atmosphérique. C'est une plante non exigeante et très résistante.

L'extrait de *Lampsana communis* utilisé dans la composition selon l'invention est obtenu par extraction hydro-glycolique de la partie aérienne de la plante. On obtient ainsi un liquide limpide de couleur brune à odeur caractéristique présentant les caractéristiques analytiques suivantes :
- teneur en eau : 18 à 26%
- pH : 6 à 8
- densité : 1.055 à 1.075
- indice de réfraction : 1.420 à 1.440

Les compositions utilisées selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc. Grâce à ces connaissances en matière de cosmétique, l'homme du métier saura quels agents de formulation ajouter aux compositions de l'invention et en quelles quantités en fonction des propriétés recherchées.

Les compositions utilisées selon l'invention peuvent se présenter sous toute forme connue de l'homme du métier dans le domaine de cosmétologie et de la dermatologie sans autre restriction galénique que l'application sur le visage et sur le corps. De façon avantageuse, les compositions selon l'invention se présentent sous la forme d'un gel, d'une lotion, d'une crème, d'une émulsion, d'un lait, d'un spray, etc.

Les exemples ci-après sont donnés à titre illustratif et ne sauraient être interprétés comme limitant la portée de l'invention. Ils concernent, d'une part, l'évaluation de l'effet anti-radicalaire du complexe *Camellia sinensis* et *Lampsana communis* vis-à-vis des kératinocytes humains en culture ainsi que l'évaluation de l'effet de ce même complexe sur le métabolisme énergétique, et d'autre part, des exemples de compositions objets de la présente invention.

Les exemples font références aux figures suivantes dans lesquelles :

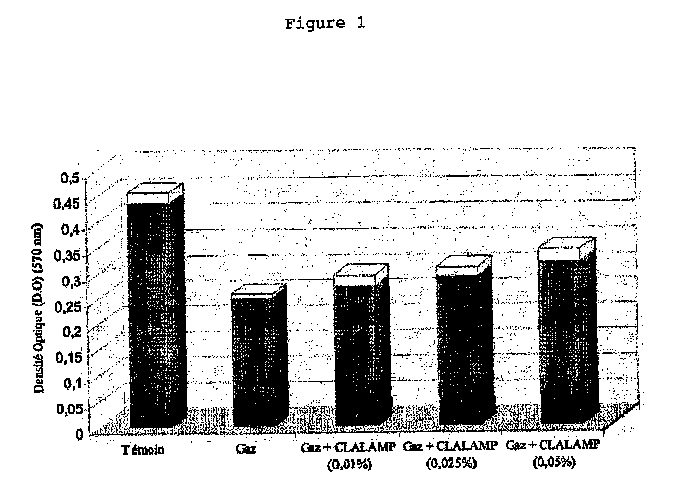

- la figure 1 représente les effets du complexe *Camellia sinensis* et *Lampsana communis* (complexe CLALAMP) sur la réversibilité de l'effet cytotoxique des gaz d'échappement vis-à-vis des kératinocytes en culture. Le complexe *Camellia sinensis* et *Lampsana communis* a été testé à 0.01%, 0.025% et 0.05%,

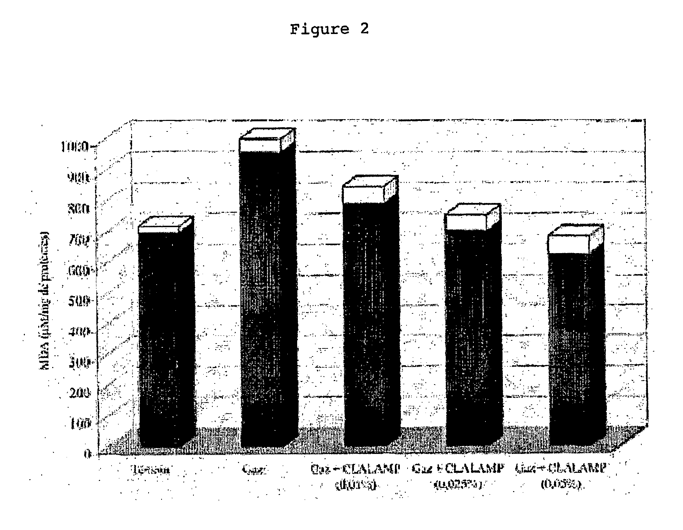

- la figure 2 représente les effets anti-radicalaires du complexe *Camellia sinensis* et *Lampsana communis* (complexe CLALAMP testé à 0.01%, 0.025% et 0.05%) par mesure du malondialdéhyde (MDA) marqueur de la lipoperoxydation sur kératinocytes en culture préalablement exposés au gaz d'échappement,

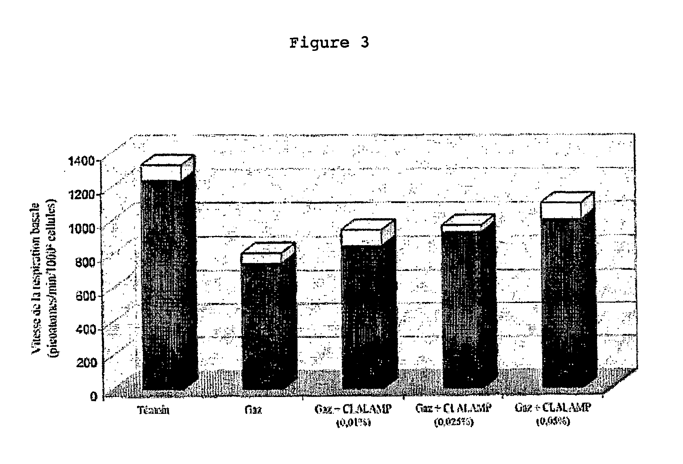

- la figure 3 représente l'effet du complexe *Camellia sinensis* et *Lampsana communis* (complexe CLALAMP testé à 0.01%, 0.025% et 0.05%) sur la vitesse de respiration des kératinocytes en culture préalablement exposés au gaz d'échappement. Les résultats sont exprimés en picoatomes d'oxygène par million de cellules et par minute,

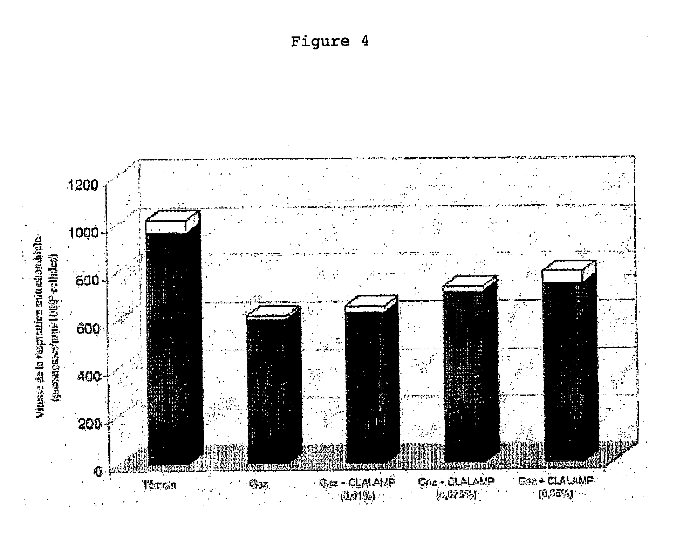

- la figure 4 représente l'effet du complexe *Camellia sinensis* et *Lampsana communis* (complexe CLALAMP testé à 0.01%, 0.025% et 0.05%) sur la vitesse de respiration mitochondriale en présence du pyruvate-malate sur kératinocytes en culture préalablement exposés au gaz d'échappement. Les résultats sont exprimés en picoatomes d'oxygène ^par million de cellules et par minute,

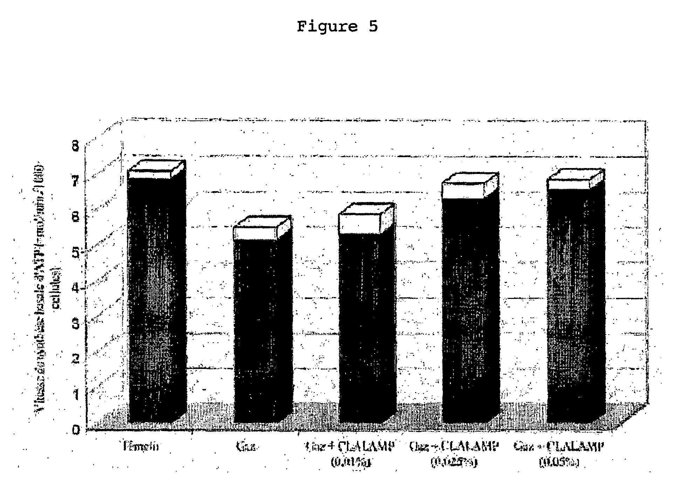

- la figure 5 représente l'effet du complexe *Camellia sinensis* et *Lampsana communis* (complexe CLALAMP testé à 0.01%, 0.025% et 0.05%) sur la synthèse d'ATP basale cellulaire de kératinocytes en culture préalablement exposés au gaz d'échappement. Les résultats sont exprimés en nmoles par million de cellules et par minutes,

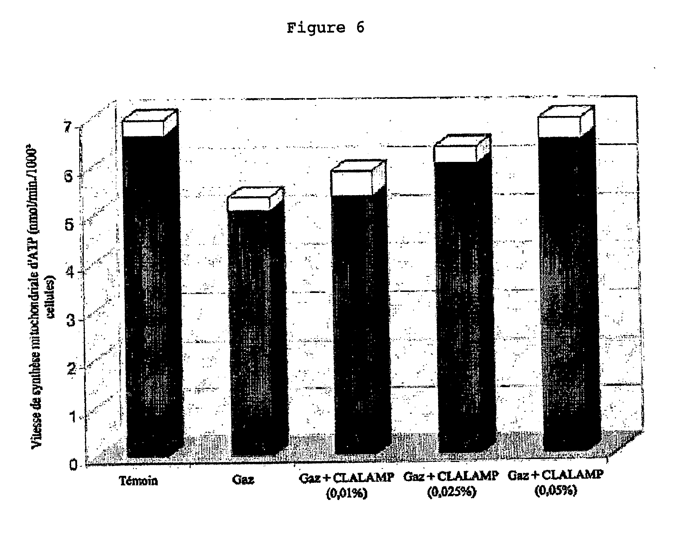

- la figure 6 représente l'effet du complexe *Camellia sinensis* et *Lampsana communis* (complexe CLALAMP testé à 0.01%, 0.025% et 0.05%) sur la vitesse de synthèse d'ATP mitochondriale cellulaire de kératinocytes en culture préalablement exposés au gaz d'échappement. Les résultats sont exprimés en nmoles par million de cellules et par minute.

### I. ÉVALUATION DE L'EFFET ANTI RADICALAIRE DU COMPLEXE Camellia sinensis et Lampsana communis VIS À VIS DES KÉRATINOCYTES HUMAINS EN CULTURE.

### A. Matériel et méthodes.

### 1. Matériel.

Les cultures de kératinocytes sont établies à partir de peau de prépuces humains récoltés lors de circoncisions et sont amplifiées dans du milieu KGM2 (Clonetics) supplémenté par l'insuline, l'EGF et de l'extrait pituitaire. Les essais ont été réalisés sur des kératinocytes entre le 2^{ème} et le 4^{ème} passage, afin d'assurer une reproductibilité entre les différentes expérimentations.

Les gaz d'échappement ont été produits par un moteur. Les gaz ont été mis au contact des kératinocytes humains en culture pendant 2 heures. Les cellules ont été par la suite incubées avec ou sans le produit à l'étude pendant 22 heures supplémentaires.

### 2. Étude de la cytotoxicité.

Cette étape a été réalisée par le Test de réduction au bleu de Formazan (MTT). Après 24 heures d'incubation des cellules en présence ou en absence du produit à l'étude à différentes concentrations, les puits contenant les cellules ont été vidés par retournement doux, puis le tapis cellulaire a été rincé avec le milieu de culture. 200 µl d'une solution de MTT diluée ont été distribués dans tous les puits. Les plaques ont été ensuite incubées à 37°C pendant 2 à 4 heures. Il y a, alors, formation de cristaux de bleu de Formazan, en quantité inversement proportionnelle à l'atteinte des succinates déshydrogénases. Les puits ont été, de nouveau, vidés par retournement doux, les cellules ont été ensuite lysées et les cristaux de bleu de Formazan dissous, par ajout de 200 µl de Diméthyl sulfoxyde (DMSO). Après homogénéisation de la coloration, par agitation, les plaques ont été lues à 570 nm à l'aide d'un spectrophotomètre.

L'essai a été conduit après 24 heures de contact du complexe *Camellia sinensis et Lampsana communis* avec les cellules.
Lot 1 : témoin négatif ne recevant aucun produit
Lot 2 : cellules exposées au gaz d'échappement
Lot 3 : cellules exposées au gaz d'échappement puis traitées avec le complexe (0,01 %)
Lot 4 : cellules exposés au gaz d'échappement puis traité avec le complexe (0,025 %)
Lot 5 : cellules exposés au gaz d'échappement puis traité avec le complexe (0,05 %)

### 3. Étude de l'activité anti-radicalaire.

L'essai a été conduit en triplicate après 24 h de contact du complexe *Camellia sinensis et Lampsana communis* avec les cellules.

### Constitution des lots

Lot 1 : témoin négatif ne recevant aucun produit
Lot 2 : cellules exposées au gaz d'échappement
Lot 3 : cellules exposées au gaz d'échappement puis traitées avec le complexe (0,01 %)
Lot 4 : cellules exposées au gaz d'échappement puis traitées avec le complexe (0,025 %)
Lot 5 : cellules exposés au gaz d'échappement puis traitées avec le complexe (0,05 %)

### a) Extraction du malondialdéhyde (MDA).

Après 24 h de contact du produit avec les cellules, ces dernières ont été remises en suspension dans :
- 250 µl de tampon Tris 50 mM, pH8 contenant NaCl 0,1M ; EDTA 20 mM
- 25 µl de SDS à 7 %
- 300 µl de HCl (0,1 N)
- 38 µl d'acide phosphotungstique à 1 % dans l'eau
- 300 µl d'acide thiobarbiturique à 0,67 % dans l'eau

Après 1 heure d'incubation dans l'obscurité à 50°C et un refroidissement dans l'eau glacée, 300 ml de n-butanol ont été ajoutés dans chaque tube. Ceux-ci ont été centrifugés à 10000 g à 0°C pendant 10 min. La phase supérieure a été récupérée pour le dosage du MDA.

### b) Dosage du malondialdéhyde (MDA).

Le MDA a été dosé par mesure de la fluorescence après séparation du complexe MDA-TBA par HPLC.
- Pompe Bischoff Model 2.200
- Injecteur automatique Alcool Model 788 autosampler
- Colonne Ultrasep C18 (30 cm x 0,18 cm) 6 mm de porosité
- Détecteur de fluorescence, jasco 821-FI

La détection de la fluorescence a été effectuée avec une excitation à 515 nm et une émission à 553 nm. L'éluent utilisé est composé de méthanol : eau, 40 : 60 (v/v) dont le pH a été ajusté avec du KOH 1 M.

La quantification a été faite par rapport à des standards traités comme les échantillons (0,125 ; 0,25 ; 0, 5 et 1 mM) à l'aide d'un logiciel informatique ICS (Pic 3) (Instrumentation, Consommable Service).

### B. RÉSULTATS.

### 1. Étude de la cytotoxicité.

Le but de cette étape était de rechercher la réversibilité de l'effet cytotoxique des gaz d'échappement après le traitement par le complexe *Camellia sinensis et Lampsana communis* vis-à-vis des kératinocytes humains en culture. Cette étude a été réalisée par la détermination de la viabilité cellulaire en utilisant le Test de MTT.

Le complexe *Camellia sinensis et Lampsana communis* incubé respectivement aux concentrations de 0,025 et 0,05% avec les kératinocytes a induit un effet significatif sur la réversibilité de la cytotoxicité induite par les gaz d'échappement. Cette inhibition de la cytotoxicité se traduit par une augmentation de la viabilité cellulaire respectivement de 16 et 27 % (Figure 1).

### 2. Étude de l'activité anti-radicalaire.

Les résultats obtenus montrent une protection significative du complexe *Camellia sinensis* et *Lampsana communis* (testé respectivement aux concentrations de 0,01, 0,025 et 0,05%) vis-à-vis de la lipoperoxydation provoquée par les gaz d'échappement. Le pourcentage de réduction de la production de MDA est respectivement de -17, -27 et -34 % (Figure 2).

### 3. Conclusions.

Dans les conditions expérimentales retenues, le complexe *Camellia sinensis* et *Lampsana communis* est apparu présenter un effet sur la réversibilité de la cytotoxicité et de l'activité pro-radicalaire induite par les gaz d'échappement au niveau des kératinocytes humains en culture après 24 heures de contact.

En effet, les dosages du MDA montrent que le complexe *Camellia sinensis* et *Lampsana communis* étudié aux concentrations de 0,01 ; 0,025 et 0,05 % a induit une protection significative des kératinocytes humains contre la lipoperoxydation provoquée par les gaz d'échappement. Le pourcentage de réduction de la production de MDA est de - 17, -27 et -34 %.

En conclusion, le complexe *Camellia sinensis* et *Lampsana communis* présente un effet anti-radicalaire significatif vis-à-vis des radicaux libres induits par les gaz d'échappement.

### II. ÉVALUATION DE L'EFFET DU COMPLEXE Camellia sinensis et Lampsana communis SUR LE MÉTABOLISME ÉNERGETIQUE.

### A. Matériel et méthodes.

### 1. Matériel.

Les cultures de kératinocytes sont établies à partir de peau de prépuces humains récoltés lors de circoncisions et sont amplifiées dans du milieu KGM2 (Clonetics) supplémenté par l'insuline, l'EGF et de l'extrait pituitaire. Les essais ont été réalisés sur des kératinocytes entre le 2^{ème} et le 4^{ème} passage, afin d'assurer une reproductibilité entre les différentes expérimentations.

Les gaz d'échappement ont été produits par un moteur. Les cellules ont été mises au contact des gaz pendant 2 heures. Elles ont été par la suite incubées avec ou sans le produit à l'étude pendant 22 heures supplémentaires.

### 2. Étude de l'effet du complexe Camellia sinensis et Lampsana communis sur la vitesse de respiration (consommation d'oxygène en picoatomes d'oxygène par million de cellules et par minute).

Cet essai a été réalisé selon 2 conditions différentes :
- effet sur la vitesse de respiration basale cellulaire au niveau des cellules non perméabilisées et en présence du glucose,
- effet sur la vitesse de la respiration mitochondriale des cellules perméabilisées en présence du substrat respiratoire, pyruvate-malate.

Cette étude a été réalisée sur des kératinocytes en culture dissociés à la trypsine. 5 à 10 millions des kératinocytes en cultures ont été mis en suspension dans 1 ml de milieu HBSS à 30°C contenant soit du glucose (respiration basale) soit du pyruvate-malate (respiration mitochondriale). La respiration a été suivie en temps réel et exprimée en picoatomes d'oxygène consommés par minute et par 10⁶ cellules. L'addition de différentes quantités du produit dans la cuve de l'oxygraphe met en évidence une éventuelle stimulation ou inhibition de la respiration.

La quantité d'oxygène dissous dans un milieu d'incubation a été déterminée à l'aide d'une électrode de Clark. L'oxygène qui diffuse à travers un film de téflon est réduit au niveau de la cathode de platine polarisée à - 0,8 Volt. Dans ces conditions, le courant passant entre cette cathode et l'anode d'argent est proportionnel à la concentration en oxygène dans la solution. Le pont ionique est assuré par une solution de KCl demi saturée. L'acquisition et le traitement des mesures sont faits sur un micro-ordinateur.

### Constitution des lots :

Lot 1 : témoin négatif ne recevant aucun produit
Lot 2 : cellules exposées au gaz d'échappement
Lot 3 : cellules exposées au gaz d'échappement puis traitées avec le complexe (0,01 %)
Lot 4 : cellules exposées au gaz d'échappement puis traitées avec le complexe (0,025 %)
Lot 5 : cellules exposées au gaz d'échappement puis traitées avec le complexe (0,05 %)

L'essai a été réalisé après 20 minutes de contact du produit le complexe *Camellia sinensis et Lampsana communis* avec les cellules.

### 3. Étude de l'effet du produit sur la synthèse d'ATP basale et mitochondriale des kératinocytes en culture.

Le but de cette étude est d'évaluer l'effet du complexe *Camellia sinensis et Lampsana communis* sur la vitesse de la synthèse d'ATP basale et mitochondriale des kératinocytes en culture. Cette détermination est réalisée par bioluminescence à l'aide du kit luciférine/luciférase.

La quantité d'ATP basale et nouvellement synthétisée dans les différents aliquotes est mesurée à travers la lumière émise lors de la réaction de consommation d'ATP suivante :

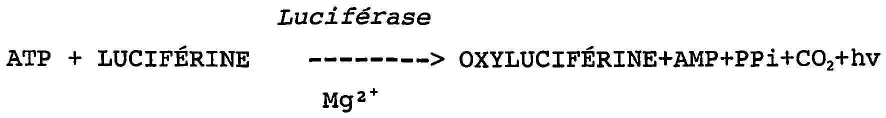

L'intensité de cette lumière émise lors de cette réaction est mesurée à l'aide d'un appareil de type Luminoscan en utilisant l'ATP monitoring reagent (ATP Bioluminescence Assay Kit HS II) de Boehringer Mannheim. Cet appareil transcrit la lumière émise lors de la réaction en RLU (unité relative de luminosité). Les RLU mesurées sont converties en moles d'ATP en se référant à une gamme étalon d'ATP.

La vitesse de synthèse d'ATP est exprimée en nmoles/min/10⁶ cellules.

Des kératinocytes en culture ont été cultivés, en étuve à CO², à raison de 10⁶ par run dans un milieu de culture ADM (Clonetics).

Le traitement consiste à appliquer de manière directe le complexe *Camellia sinensis et Lampsana communis* à la concentration désirée sur les cellules en suspension dans la cuve de l'oxygraphe. Les cellules à la concentration de 10⁶ cellules/ml sont mises en suspension dans un "tampon respiratoire" (Hanks-Hepes glucose 20 mM), dans la cuve de l'oxygraphe thermostatée à 30°C et agitée. Les cellules sont perméabilisées à l'aide de digitonine. L'ajout d'un substrat respiratoire (pyruvate 10 mM et malate 10 mM) permet d'observer une vitesse de consommation d'oxygène (état 2 selon Chance). Après l'ajout de différentes quantités du produit (concentrations finales : 0,01 ; 0,025 et 0,05 %) dans la cuve de l'oxygraphe et à intervalles réguliers, un aliquote est prélevé dans la cuve de l'oxygraphe pour y doser l'ATP selon la méthode décrite ci-dessus. L'addition de différentes quantités du produit dans la cuve de l'oxygraphe permet ainsi de mettre en évidence une éventuelle activation ou inhibition de la synthèse d'ATP.

### Constitution des lots :

Lot 1 : témoin négatif ne recevant aucun produit
Lot 2 : cellules exposées au gaz d'échappement
Lot 3 : cellules exposées au gaz d'échappement puis traitées avec le complexe (0,01 %)
Lot 4 : cellules exposées au gaz d'échappement puis traitées avec le complexe (0,025 %)
Lot 5 : cellules exposées au gaz d'échappement puis traitées avec le complexe (0,05 %)

### B. RÉSULTATS.

### 1. Étude de l'effet du complexe à différentes doses sur la vitesse de respiration (consommation d'oxygène) des kératinocytes en culture.

Cet essai a été réalisé selon 2 conditions expérimentales différentes. Les résultats sont exprimés en picoatomes d'oxygène par million de cellules et par minute.

Le complexe *Camellia sinensis et Lampsana communis,* incubé respectivement aux concentrations de 0,01, 0,025 et 0,05% avec les kératinocytes préalablement exposés aux gaz d'échappement, a induit un effet significatif, sur la vitesse de consommation d'oxygène aux concentrations étudiées. Cette stimulation de la respiration basale se traduit par une augmentation sensible de la vitesse de consommation d'oxygène respectivement de 14, 24 et 34 % (Figure 3).

### 2. Effet du complexe sur la vitesse de la respiration mitochondriale.

Cet essai a été réalisé sur les cellules perméabilisées en présence du substrat respiratoire, pyruvate-malate.

Le complexe *Camellia sinensis et Lampsana communis,* incubé aux différentes concentrations avec les kératinocytes préalablement exposés aux gaz d'échappement, a induit un effet significatif, sur la vitesse de consommation d'oxygène aux concentrations de 0,025 et 0,05 %. Cette stimulation de la respiration mitochondriale se traduit par une augmentation sensible de la vitesse de consommation d'oxygène respectivement de 19 et 24 % (Figure 4).

### 3. Effet du complexe à différentes doses sur la synthèse d'ATP basale et mitochondriale des kératinocytes en culture.

Cet essai a été réalisé selon 2 conditions expérimentales différentes. Les résultats sont exprimés en nmoles par minute et par million de cellules.

### a) Effet sur la synthèse d'ATP basale cellulaire.

Cet essai a été effectué sur des cellules entières non perméabilisées en présence du glucose.

Le complexe *Camellia sinensis et Lampsana communis,* incubé aux différentes concentrations avec les kératinocytes préalablement exposés aux gaz d'échappement, a induit un effet significatif sur la vitesse de synthèse d'ATP basale aux concentrations de 0,025 et 0,05%. La vitesse de synthèse d'ATP basale augmente en effet respectivement de 23 et 29 % (Figure 5).

### b) Effet sur la vitesse de synthèse d'ATP mitochondriale cellulaire.

Cet essai a été effectué sur des cellules entières perméabilisées en présence du pyruvate-malate.

Le complexe *Camellia sinensis et Lampsana communis,* incubé aux différentes concentrations avec les kératinocytes préalablement exposés aux gaz d'échappement, a induit un effet significatif sur la vitesse de synthèse mitochondriale d'ATP aux concentrations de 0,025 et 0,05 %. La vitesse de synthèse d'ATP mitochondriale augmente en effet respectivement de 19 et 29 % (Figure 6).

### III. CONCLUSIONS SUR LES RÉSULTATS OBTENUS.

Dans les conditions expérimentales, au vu des résultats obtenus le complexe *Camellia sinensis et Lampsana communis* a induit un effet :
- significatif sur la réversibilité de la cytotoxicité induite par les gaz d'échappement qui est respectivement de 16 et 27 % pour des concentrations de complexe *Camellia sinensis* et *Lampsana communis* de 0,025 et 0,05 % (Figure 1),
- significatif sur la vitesse de la respiration basale qui est respectivement de 24 et 34 % pour des concentrations de complexe *Camellia sinensis* et *Lampsana communis* de 0,025 et 0,05 % (Figure 3),
- significatif sur la vitesse de la respiration mitochondriale qui est respectivement de 19 et 24 % pour des concentrations de complexe *Camellia sinensis* et *Lampsana communis* de 0,025 et 0,05 % (Figure 4),
- significatif sur la vitesse de synthèse d'ATP basale qui est respectivement de 23 et 29 % pour des concentrations de complexe *Camellia sinensis* et *Lampsana communis* de 0,025 et 0,05 % (Figure 5),
- significatif sur la vitesse de synthèse d'ATP mitochondriale qui est respectivement de 19 et 29 % pour des concentrations de complexe *Camellia sinensis* et *Lampsana communis* de 0,025 et 0,05 % (Figure 6).

On notera, que de manière surprenante, le complexe *Camellia sinensis et Lampsana communis* est actif à des concentrations très faibles, signe de la synergie entre les deux extraits.

### IV. EXEMPLES DE COMPOSITIONS UTILISABLE SELON L'INVENTION.

| A. GEL CRÈME ANTI-POLLUTION. | |
|---|---|
| EAU DÉMINÉRALISÉE | q.s.p 100 |
| PEMULEN TR1 | 0,5 |
| GLYCÉRINE | 5,0 |
| SEPIGEL 305 | 1,0 |
| EXTRAIT DE *Camellia sinensis* | 0,05 |
| EXTRAIT DE *Lampsana communis* | 0,05 |
| ISONONYL ISONONANOATE | 7,0 |
| C12 - C15 ALKYL BENZOATE | 3,0 |
| HUILE DE SILICONE | 2,0 |
| HYDROXYDE DE SODIUM | 0,2 |
| PARFUM | 0,3 |
| COLORANTS A 1 % | 0,12 |
| CONSERVATEURS | 1,0 |
| | |

| B. CRÈME ANTI-POLLUTION. | |
|---|---|
| EAU DÉMINÉRALISÉE | q.s.p 100 |
| CETEARYL GLUCOSIDE | 5,0 |
| TRIGLYCÉRIDES C8-C10 | 10 |
| HUILE DE SILICONE | 2,0 |
| SILICONE VOLATILE | 5,0 |
| CARBOMÈRE | 0,2 |
| GLYCÉRINE | 5,0 |
| EXTRAIT DE *Camellia sinensis* | 0,05 |
| EXTRAIT DE *Lampsana communis* | 0,05 |
| PARFUM | 0,3 |
| CONSERVATEURS | 1,0 |

## Revendications

1. Utilisation d'une composition cosmétique contenant un extrait de *Camellia sinensis* et un extrait de *Lampsana communis* pour augmenter la vitesse de synthèse d'ATP basale et mitochondriale des cellules de la peau, ladite composition contenant de 0,025 à 5 % en poids d'extrait de *Camellia sinensis* et d'extrait de *Lampsana communis.*

2. Utilisation selon la revendication 1, dans laquelle l'extrait de *Camellia sinensis* est un extrait hydro-glycolique des feuilles de cette plante.

3. Utilisation selon la revendication 1 ou 2, dans laquelle l'extrait de *Lampsàna communis* est un extrait hydro-glycolique de la partie aérienne de cette plante.

4. Utilisation selon l'une quelconque des revendications 1 à 3, dans laquelle la composition comprend en outre un ou plusieurs agents de formulation ou additifs tels que des adoucissants, des colorants, des agents filmogènes, des tensio-actifs, des parfums, des conservateurs, des émulsionnants, des huiles, des glycols, des vitamines et des filtres UV.

## Claims

1. Use of a cosmetic composition comprising a *Camellia sinensis* extract and a *Lapsana communis* extract for increasing the basal and mitochondrial ATP synthesis rate of the skin cells, said composition comprising from 0.025 to 5% by weight of the *Camellia sinensis* extract and *Lapsana communis* extract.

2. Use according to claim 1, wherein *Camellia sinensis* extract is an hydroglycolic extract from the leaves of this plant.

3. Use according to claim 1 or 2, wherein *Lapsana communis* extract is an hydroglycolic extract from the aerial part of this plant.

4. Use according to anyone of claims 1 to 3, wherein the composition further comprises one or more formulation agents or additives such as demulcents, colorants, film-forming agents, surface-active agents, perfumes, preservatives, emulsifiers, oils, glycols, vitamins and UV filters.

## Patentansprüche

1. Verwendung einer kosmetischen Zusammensetzung, die zur Erhöhung der basalen und mitochondrialen ATP Synthesegeschwindigkeit von Hautzellen einen *Camellia sinensis*-Extrakt und einen *Lampsana communis*-Extrakt enthält, wobei besagte Zusammensetzung von 0,025 bis 5 Gewichtsprozenten von dem *Camellia sinensis-* Extrakt und von dem *Lampsana communis*-Extrakt enthält.

2. Verwendung nach Anspruch 1, wobei der *Camellia sinensis*-Extrakt ein hydroglykolischer Extrakt aus den Blättern dieser Pflanze ist.

3. Verwendung nach Anspruch 1 oder 2, wobei der *Lampsana communis-*Extrakt ein hydroglykolischer Extrakt des oberirdischen Teils dieser Pflanze ist.

4. Verwendung nach einem beliebigen der Ansprüche 1 bis 3, wobei die Zusammensetzung des Weiteren einen oder mehrere Formulierungsmittel oder Zusatzstoffe enthält, wie Weichmacher, Farbstoffe, filmbildende Stoffe, oberflächenaktive Stoffe, Duftstoffe, Konservierungsstoffe, Emulgatoren, Öle, Glykole, Vitamine und UV-Filter.
